# EUROPEAN PATENT SPECIFICATION

(11) **EP 2 295 057 B1**
(45) Date of publication and mention of the grant of the patent: **23.01.2013**
(21) Application number: 10183680.7
(22) Date of filing: 03.12.2002
(51) Int. Cl.: A61K 31/44, A61K 31/535, A61K 31/65, A61K 31/435, A61K 31/505, A61K 31/47, A61P 35/00

(54) **Aryl urea compounds in combination with other cytostatic or cytotoxic agents for treating human cancers**
Arylharnstoff-Verbindungen in Kombination mit anderen zytostatisch oder zytotoxisch wirksamen Stoffen zur Behandlungen menschlicher Krebserkrankungen
Composés de type urée aryle combines à d'autres agents cytostatiques ou cytotoxiques et servant à traiter des cancers humains

(30) Priority: 03.12.2001 US 334609 P
(43) Date of publication of application: 16.03.2011
(62) Divisional of application: 06023696.5
(73) Proprietor: Bayer HealthCare LLC, Tarrytown, NY 10591 (US)
(72) Inventor: Carter, Christopher A., Guilford, CT 06437 (US); Gibson, Neil, Melville, NY 11747 (US); Hibner, Barbara, Madison, CT 06443 (US); Humphrey, Rachel W., New York, NY 10128 (US); Trail, Pamela, Madison, CT 06443 (US); Vincent, Patrick, Encinitas, CA 92024 (US); Zhai, Yifan, Rockville, MD 20850 (US)
(74) Representative: Weiss, Wolfgang

(56) References cited:
- WO-A-00/42012
- STRUMBERG DIRK ET AL: "Phase I and pharmacokinetic study of the Raf kinase inhibitor bay 43-9006 in patients with locally advanced or metastatic cancer.", PROCEEDINGS OF THE AMERICAN ASSOCIATION FOR CANCER RESEARCH ANNUAL, vol. 42, March 2001 (2001-03), page 543, XP001145481, 92nd Annual Meeting of the American Association for Cancer Research;New Orleans, LA, USA; March 24-28, 2001, March, 2001 ISSN: 0197-016X
- GEIGER T ET AL: "Antitumor activity of a C-raf antisense oligonucleotide in combination with standard chemotherapeutic agents against various human tumors transplanted subcutaneously into nude mice.", CLINICAL CANCER RESEARCH: AN OFFICIAL JOURNAL OF THE AMERICAN ASSOCIATION FOR CANCER RESEARCH. UNITED STATES JUL 1997, vol. 3, no. 7, July 1997 (1997-07), pages 1179-1185, XP001145779, ISSN: 1078-0432
- SPENCE R A ET AL: "Unresectable fibrolamellar hepatocellular carcinoma treated with intra-arterial lipiodolised doxorubicin. A case report.", SOUTH AFRICAN MEDICAL JOURNAL = SUID-AFRIKAANSE TYDSKRIF VIR GENEESKUNDE 21 NOV 1987 LNKD- PUBMED:2825364, vol. 72, no. 10, 21 November 1987 (1987-11-21), pages 701-703, XP009143227, ISSN: 0256-9574
- SORBERA L A ET AL: "Bay-43-9006. Oncolytic, Raf kinase inhibitor", DRUGS OF THE FUTURE, PROUS SCIENCE, ES, vol. 27, no. 12, 1 December 2002 (2002-12-01), pages 1141-1147, XP002581752, ISSN: 0377-8282

## Description

### Field of the Invention

This invention relates to aryl urea compounds in combination with doxorubicin and their use in treating raf kinase mediated diseases such as hepatocellular cancer.

### Background of the Invention

The p21 oncogene, ras, is a major contributor to the development and progression of human solid cancers and is mutated in 30% of all human cancers (Bolton et al. Ann. Re. Med. Chem. 1994, 29, 165-174; Bos. Cancer Res. 1989, 49, 4682-9). In its normal, unmutated form, the ras protein is a key element of the signal transduction cascade directed by growth factor receptors in almost all tissues (Avruch et al. Trends Biochem. Sci. 1994, 19, 279-83). Biochemically, ras is a guanine nucleotide binding GTPase protein that cycles between a GTP-bound activated and a GDP-bound inactive form. It's endogenous GTPase activity is strictly self-regulated and is also controlled by other regulatory proteins. The endogenous GTPase activity of mutations is reduced. Therefore, the protein delivers constitutive growth signals to downstream effectors such as the enzyme raf kinase. This leads to the cancerous growth of the cells which carry these mutants (Magnuson et al. Semin. Cancer Biol. 1994, 5, 247-53). It has been shown that inhibiting the effect of active ras by inhibiting the raf kinase signaling pathway via administration of deactivating antibodies to raf kinase or by co-expression of dominant negative raf kinase or dominant negative MEK, the substrate of raf kinase, leads to the reversion of transformed cells to the normal growth phenotype (see: Daum et al. Trends Biochem. Sci. 1994, 19, 474-80; Friedman et al. J. Biol. Chem. 1994, 269, 30105-8; Kocj et al. Nature 1991, 349, 426-28). These references have further indicated that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human cancer types (Monia et al., Nat. Med. 1996, 2, 668-75).

Therefore, compounds which can act as raf kinase inhibitors represent an important group of chemotherapeutic agents for use in the treatment of a variety of different cancer types.

Strumberg et al. (Proceedings of the American Association for Cancer Research Annual, 42, 2001, 543) discloses a pharmacokinetic study of sorafenib (N-(4-chloro-3-(trifluoromethyl)phenyl-N-(4-(2-(N-methylcarbamoyl)-4-pyridyloxy)phenyl)urea) in patients with locally advanced metastatic cancer.

WO 00/42012 discloses ω-carboxyaryl substituted diphenyl ureas as raf kinase inhibitors and their use in solid cancers.

### Summary of the Invention

The present invention is directed to the use of an aryl urea compound which is a tosylate salt of N-(4-chloro-3-(trifluoromethyl)phenyl-N'-(4-(2-(N-methylcarbamoyl)-4-pyridyloxy)phenyl)urea and doxorubicin or a pharmaceutically acceptable salt thereof for the preparation of a medicament for the treatment of hepatocellular cancer.

Generally, cytotoxic and/or cytostatic agents may be combined with aryl urea compound raf kinase inhibitors which will serve to (1) yield better efficacy in reducing the growth of a tumor or even eliminate the tumor as compared to administration of either agent alone, (2) provide for the administration of lesser amounts of the chemotherapeutic agents, (3) provide for a chemotherapeutic treatment that is well tolerated in the patient with fewer deleterious pharmacological complications than observed with single agent chemotherapies and certain other combined therapies, (4) provide for treating a broader spectrum of different cancer types in mammals, especially humans, (5) provide for a higher response rate among treated patients, (6) provide for a longer survival time among treated patients compared to standard chemotherapy treatments, (7) provide a longer time for tumor progression, and/or (8) yield efficacy and tolerability results at least as good as those of the agents used alone, compared .to known instances where other cancer agent combinations produce antagonistic effects.

### Brief Description of the Figures

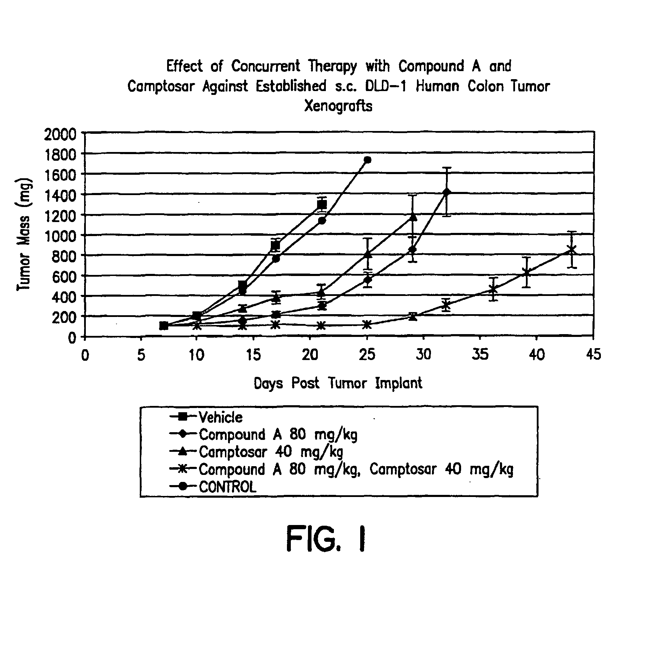

Figure 1 shows the response of established s.c. DLD-1 human colon tumor xenografts to Compound A and Camptosar alone and in combination.

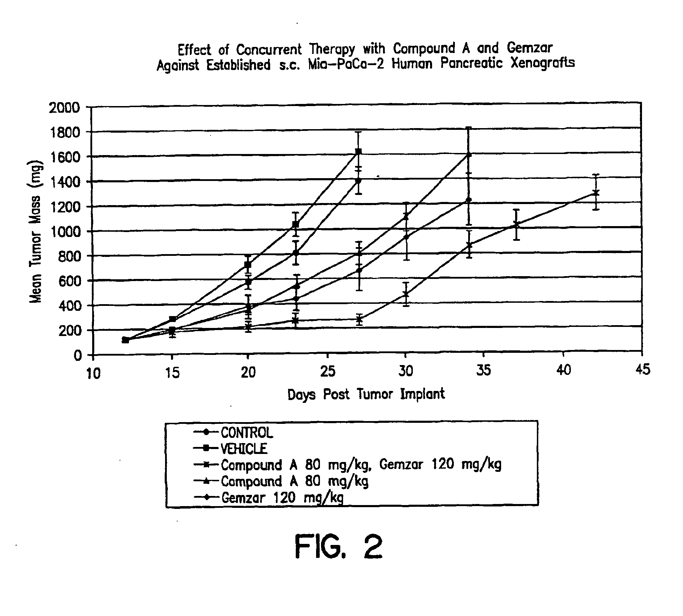

Figure 2 shows the response of established s.c. MiaPaCa-2 human pancreatic tumor xenografts to Compound A and Gemzar alone and in combination.

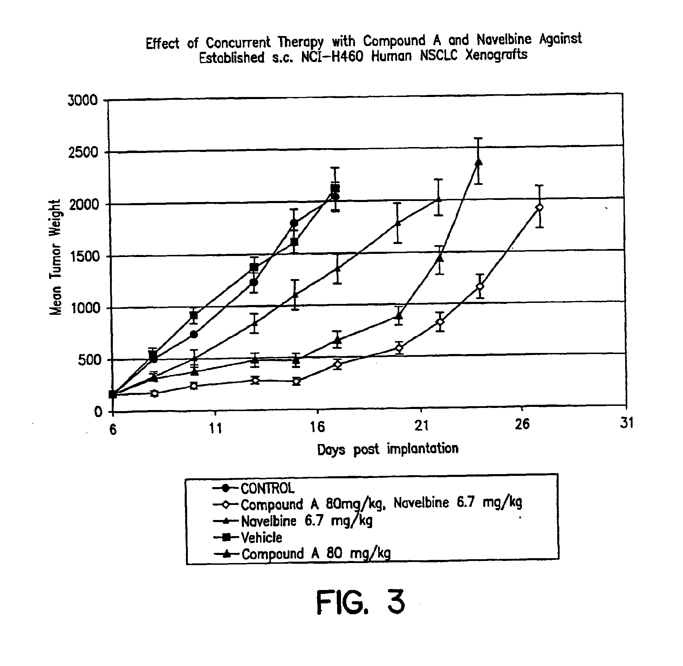

Figure 3 shows the response of established s.c. NCI-H460 human NSCLC tumor xenografts to Compound A and Navelbine alone and in combination.

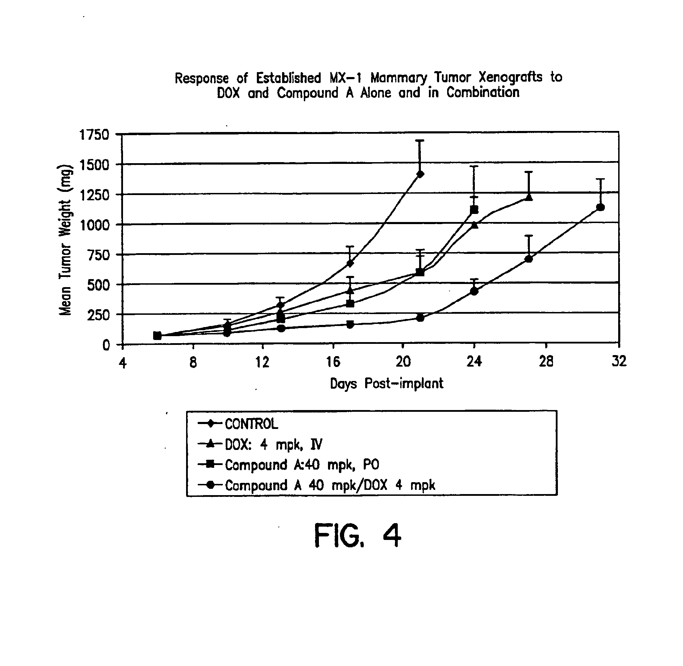

Figure 4 shows the response of established MX-1 mammary tumor xenografts to Compound A and DOX alone and in combination.

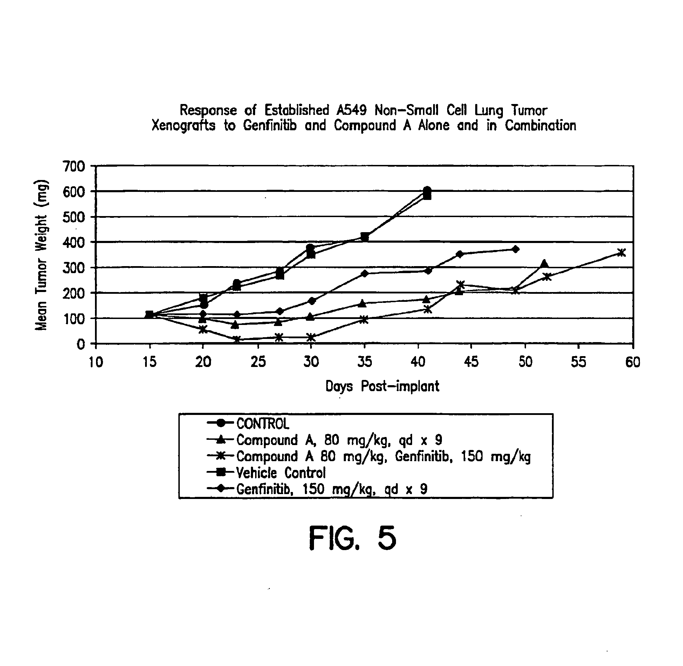

Figure 5 shows the response of established A549 non-small cell hmg tumor xenografts to Compound A and Gefinitib alone and in combination.

### Detailed Description of the Invention

The present invention relates to a combination comprising tosycate salt of soragenib with at least one other chemotherapeutic (a) cytotoxic agent or (b) cytostatic agent which is doxorubicin or pharmaceutically acceptable salts of any component.

A combination of a cytotoxic or cytostatic agent and (1) a substituted bridged aryl urea compound, or (2) a substituted bridged aryl urea compound having at least one bridged aryl urea structure with substituent(s) on the remote ring, or (3) a γ-carboxyamide substituted bridged aryl urea compound, or (4) a compound or a pharmaceutically acceptable salt of a compound of formula I

A-D-B (I)

is described

In formula I, D is -NH-C(O)-NH-,
A is a substituted moiety of up to 40 carbon atoms of the formula: -L-(M-L¹)_{q}, where L is a 5 or 6 membered cyclic structure bound directly to D, L¹ comprises a substituted cyclic moiety having at least 5 members, M is a bridging group having at least one atom, q is an integer of from 1-3; and each cyclic structure of L and L¹ contains 0-4 members of the group consisting of nitrogen, oxygen and sulfur, and
B is a substituted or unsubstituted, up to tricyclic aryl or heteroaryl moiety of up to 30 carbon atoms with at least one 6-member cyclic structure bound directly to D containing 0-4 members of the group consisting of nitrogen, oxygen and sulfur,
wherein L¹ is substituted by at least one substituent selected from the group consisting of -SO₂Rₓ, -C(O)Rₓ and -C(NR_{y}) R_{z},
Ry is hydrogen or a carbon based moiety of up to 24 carbon atoms optionally containing heteroatoms selected from N, S and O and optionally halosubstituted, up to per halo,
R_{z} is hydrogen or a carbon based moiety of up to 30 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen;
Rₓ is R_{z} or NRₐR_{b} where Rₐ and R_{b} are
a) independently hydrogen,
   a carbon based moiety of up to 30 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen, or
   -OSi(R_{f})₃ where R_{f} is hydrogen or a carbon based moiety of up to 24 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen; or
b) Rₐ and R_{b} together form a 5-7 member heterocyclic structure of 1-3 heteroatoms selected from N, S and O, or a substituted 5-7 member heterocyclic structure of 1-3 heteroatoms selected from N, S and O substituted by halogen, hydroxy or carbon based substituents of up to 24 carbon atoms, which optionally contain heteroatoms selected from N, S and O and are optionally substituted by halogen; or
c) one of Rₐ or R_{b} is -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, and carbon based substituents of up to 24 carbon atoms, which optionally contain heteroatoms selected from N, S and O and are optionally substituted by halogen;
where B is substituted, L is substituted or L¹ is additionally substituted, the substituents are selected from the group consisting of halogen, up to per-halo, and Wn, where n is 0-3;
wherein each W is independently selected from the group consisting of -CN, -CO₂R⁷, -C(O)NR⁷R⁷, -C(O)-R⁷, -NO₂, -OR⁷, -SR⁷, -NR⁷R⁷, -NR⁷C(O)OR⁷, - NR⁷C(O)R⁷, -Q-Ar, and carbon based moieties of up to 24 carbon atoms, optionally containing heteroatoms selected from N, S and O and optionally substituted by one or more substituents independently selected from the group consisting of -CN, -CO₂R⁷, - C(O)R⁷, -C(O)NR⁷R⁷, -OR⁷, -SR⁷, -NR⁷R⁷, -NO₂, -NR⁷C(O)R⁷, -NR⁷C(O)OR⁷ and halogen up to per-halo; with each R⁷ independently selected from H or a carbon based moiety of up to 24 carbon atoms, optionally containing heteroatoms selected from N, S and O and optionally substituted by halogen,
wherein Q is -O-, -S-, -N(R⁷)-, -(CH₂)ₘ-, -C(O)-, -CH(OH)-, -(CH₂)ₘO-, - (CH₂)ₘS-, -(CH₂)ₘN(R⁷)-, -O(CH₂)ₘ- CHX^{a}-, -CX^{a}₂-, -S-(CH₂)ₘ- and -N(R⁷)(CH₂)ₘ-, where m= 1-3, and X^{a} is halogen; and
Ar is a 5- or 6-member aromatic structure containing 0-2 members selected from the group consisting of nitrogen, oxygen and sulfur, which is optionally substituted by halogen, up to per-halo, and optionally substituted by Zₙ₁, wherein n1 is 0 to 3 and each Z is independently selected from the group consisting of -CN, -CO₂R⁷, -C(O)R⁷, -C(O)NR⁷R⁷, -NO₂, -OR⁷, - SR⁷ -NR⁷R⁷, -NR⁷C(O)OR⁷, -NR⁷C(O)R⁷, and a carbon based moiety of up to 24 carbon atoms, optionally containing heteroatoms selected from N, S and O and optionally substituted by one or more substituents selected from the group consisting of -CN, -CO₂R⁷, -COR⁷, -C(O)NR⁷R⁷, -OR⁷, -SR⁷, -NO₂, -NR⁷R⁷, -NR⁷C(O)R⁷, and -NR⁷C(O)OR⁷, with R⁷ as defined above.

In formula I, suitable hetaryl groups include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-oxadiazol-3- or -5-yl, 1,3,4-thiadiazol-2- or -5-yl, 1,3,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8-isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yi.

Suitable alkyl groups and alkyl portions of groups, e.g., alkoxy, etc. throughout include methyl, ethyl, propyl, butyl, etc., including all straight-chain and branched isomers such as isopropyl, isobutyl, *sec*-butyl, *tert*-butyl, etc.

Suitable aryl groups which do not contain heteroatoms include, for example, phenyl and 1- and 2 -naphthyl.

The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl", as used herein also includes saturated heterocyclic groups.

Suitable halogen groups include F, Cl, Br, and/or I, from one to per-substitution (i.e. all H atoms on a group replaced by a halogen atom) being possible where an alkyl group is substituted by halogen, mixed substitution of halogen atom types also being possible on a given moiety.

A pharmaceutical preparation is described which comprises (1) quantities of (a) an aryl urea compound e.g., Compound A (defined below) and (b) at least one other cytotoxic or cytostatic agent in amounts which are jointly effective for treating a cancer, where any component (a) or (b) can also be present in the form of a pharmaceutically acceptable salt if at least one salt-forming group is present, with (2) one or more pharmaceutically acceptable carrier molecules.

Treating a cancer that can be treated by administration of an aryl urea compound that targets raf kinase, and at least one other chemotherapeutic agent which is a cytotoxic or cytostatic agent is described. The aryl urea compound and cytotoxic or cytostatic agent are administered to a mammal in quantities which together are therapeutically effective against proliferative diseases, including but not limited to colon, gastric, lung, pancreatic, ovarian, prostate, leukemia, melanoma, hepatocellular, renal, head and neck, glioma, and mammary cancers. Thus, the aryl urea compound is effective for raf kinase-mediated cancers. However, these compounds are also effective for cancers not mediated by raf kinase.

The cytotoxic or cytostatic agent includes but is not limited to irinotecan, vinorelbine, gemcitabine, gefinitib, paclitaxel, taxotere, doxorubicin, cisplatin, carboplatin, BCNU, CCNU, DTIC, melphalan, cyclophosphamide, ara A, ara C, etoposide, vincristine, vinblastine, actinomycin D, 5-fluorouracil, methotrexate, herceptin, and mitomycin C.

Treating a cancer in a mammal, especially a human patient, comprising administering an aryl urea compound in combination with a cytotoxic or cytostatic chemotherapeutic agent including but not limited to DNA topoisomerase I and II inhibitors, DNA intercalators, alkylating agents, microtubule disruptors, hormone and growth factor receptor agonists or antagonists, other kinase inhibitors and antimetabolites, is described.

Treating a cancer in a mammal, especially a human patient, comprising administering an aryl urea compound in combination with irinotecan is described.

Treating a cancer in a mammal, especially a human patient, comprising administering an aryl urea compound in combination with paclitaxel is described.

Treating a. cancer in a mammal, especially a human patient, comprising administering an aryl urea compound in combination with vinorelbine is described.

Treating a cancer in a mammal, especially a human patient, comprising administering an aryl urea compound in combination with gefinitib is described.

Treating a cancer in a mammal, especially a human patient, comprising administering an aryl urea compound in combination with doxorubicin is described.

Treating a cancer in a mammal, especially a human patient, comprising administering an aryl urea compound in combination with gemcitabine is described.

A variety of human cancers, including but not limited to pancreatic, lung, colon, ovarian, prostate, leukemia, melanoma, hepatocellular, renal, head and neck, glioma, and mammary carcinomas can be treated.

In preferred embodiment, a method is disclosed for administering the chemotherapeutic agents, including the aryl urea compounds and the cytotoxic and cytostatic agents, to the patient by oral delivery or by intravenous injection or infusion.

In another preferred embodiment, the composition comprising the aryl urea compound or the cytotoxic or cytostatic agent can be administered to a patient in the form of a tablet, a liquid, a topical gel, an inhaler or in the form of a sustained release composition.

In one embodiment of the invention, the aryl urea compound can be administered simultaneously with doxorubicin to a patient with a cancer, in the same formulation or, more typically in separate formulations and, often, using different administration routes. Administration can also be sequentially, in any order.

In a preferred embodiment, the aryl urea compound can be administered in tandem with doxorubicin wherein the aryl urea compound can be administered to a patient once or more per day for up to 28 consecutive days with the concurrent or intermittent administration of a cytotoxic or cytostatic agent over the same total time period.

In another preferred embodiment of the invention, the aryl urea compound can be administered to a patient at an oral, intravenous, intramuscular, subcutaneous, or parenteral dosage which can range from about 0.1 to about 300 mg/kg of total body weight.

In another preferred embodiment, doxorubicin can be administered to a patient at an intravenous, intramuscular, subcutaneous, or parenteral dosage which can range from about 0.1 mg to 300 mg/kg of patient body weight.

According to the invention the aryl urea compound is a tosylate salt of N-(4-chloro-3-(trifluoromethyl)phenyl)-N'-(4-(2-(N-methylcarbamoyl)-4-pyridyloxy)phenyl)urea. The scaleable synthesis of the aryi urea compound is disclosed in Organic Process Research and Development (2002), Vol.6, Issue #6, 777-781, and copending patent application serial no. 09/948,915 filed September 10, 2001 which we incorporated herein by reference.

Further, .the invention relates to a method of inhibiting proliferation of cancer cells comprising contacting cancer cells with a pharmaceutical preparation or product of the invention, especially a method of treating a proliferative disease comprising contacting a subject, cells, tissues or a body fluid of said subject, suspected of having a cancer with a pharmaceutical composition or product of this invention.

This invention also relates to compositions containing both the aryl urea compound and the doxorubicins, in the amounts of this invention. This invention further relates its comprising separate doses of the two mentioned chemotherapeutic agents in separate containers. The combinations are described can also be formed in vivo, e.g., in a patient's body.

The term "cytotoxic" refers to an agent which can be administered to kill or eliminate a cancer cell The term "cytostatic" refers to an agent which can be administered to restrain tumor proliferation rather than induce cytotoxic cytoreduction yielding an elimination of the cancer cell from the total viable cell population of the patient. The chemotherapeutic agents described herein, e.g., irinotecan, vinorelbine, gemcitabine, doxorubicin, and paclitaxel are considered cytotoxic agents. Gefinitib is considered a cytostatic agent. These cytotoxic and cytostatic agents have gained wide spread use as chemotherapeutics in the treatment of various cancer types and are well known.

Irinotecan (CPT-11) is sold under the trade name of Camptosar^{®} by Pharmacia & Upjohn Co., Kalamazoo, MI. Irinotecan is a camptothecin or topoisomerase I inhibitor. While not being bound by a theory, it is believed that by blocking this enzyme in cells, damage occurs when the cell replicates, and the cancer growth is thus controlled. The cytotoxic effect is believed due to double-stranded DNA damage produced during DNA synthesis when replication enzymes interact with the tertiary complex formed by topoisomerase I, DNA, and either Irinotecan or SN-38 (its active metabolite). Conversion of irinotecan to SN-38 is believed to occur in the liver. Irinotecan is typically administered by injection or via i.v. infusion.

Vinorelbine (Vinorelbine tartrate) has the molecular formula C₄₅H₅₄N₄O_{8·}2C₄H₆O₆ with a molecular weight of 1079.12 and is sold under the tradename of Navelbine^{®} by Glaxo SmithKline, Research Triangle Park. Vinorelbine is a semi-synthetic vinca alkaloid with antitumor activity. The chemical name is 3',4'-didehydro-4'deoxy-C-norvincaleukoblastine [R-(R,R)-2,3-dihydroxybutanedioate (1:2)(salt)]. While not bound by a theory, the antitumor activity of vinorelbine is believed to be due primarily to inhibition of mitosis at the metaphase stage through its interaction with tubulin. Vinorelbine may also interfere with: 1) amino acid, cyclic AMP, and glutathione metabolism, 2) calmodulin dependent Ca++ transport ATPase activity, 3) cellular respiration, and 4) nucleic acid and lipid biosynthesis. Vinorelbline is typically administered by intravenous injection (i.v.) or by other appropriate infusion techniques. Vinorelbine is typically prepared in normal saline, D5W or other compatible solutions.

Gemcitabine is sold under the trade name Gemzar^{®} (Eli Lilly & Co., Indianapolis, IN). Gemzar is an antimetabolite related to cytarabine. Gemzar^{®} is indicated for patients previously treated with 5-fluorouracil. Gemzar^{®} is a pyrimidine analog that has a broad range of activity against solid tumors including but not limited to breast, ovarian, pancreatic, and lung carcinomas. It is believed to be incorporated into DNA of fast growing cancer cells, affecting replication. Gemzar^{®} is a nucleoside analogue which disrupts DNA synthesis in S-phase cells and blocks the progression of cells through the G1/S phase boundary. Gemcitabine HCl is believed to be metabolized by nucleoside kinases to active diphosphate and triphosphate forms which inhibit ribonucleotide reductase and which competes with CTP for incorporation into DNA, respectively. Gemzar^{®} is administered by intravenous injection (i.v.) or by other appropriate infusion techniques.

Gefinitib is sold under the tradename Iressa^{®} (ZD 1839, Astra-Zeneca). Iressa is a 4-anilinoquinazoline and is believed to inhibit kinase acitivity of the epidermal growth factor regulator (EGFR). Mechanism of action studies seem to indicate that Iressa is an ATP-competitive inhibitor of EGFR and blocks autophosphorylation of the receptor when the receptor is stimulated by binding EGF or TGFα. Iressa is orally bioavailable and has demonstrated preclinal efficacy against tumor models that simultaneously express EGFR and one of its ligands, TGFα. Iressa has also been shown to inhibit the in vitro proliferation of cell lines that overexpress either EGFR or Her2. In clinical trials, Iressa has been maintained p.o. on a continuous daily schedule at up to 800 mg/day.

Doxorubicin (DOX) is sold under the tradename Adriamycin^{®} (Adria). DOX is an anthracylcine that is believed to intercalate in DNA and interact with DNA Topoisomerase II to induce double-stranded DNA breaks. DOX exhibits a broad spectrum of anti-tumor efficacy. DOX is clinically administered intravenously on an intermittent schedule. The primary route of elimination of DOX is through the bile with no enterohepatic circulation. The dose-limiting acute toxicity of DOX is myelosuppresion. Other common, but not usually dose-limiting toxicities are gastrointestinal, alopecia, and local tissue damage/ulceration at the injection site due to extravasation of the drug.

Paclitaxel is sold under the tradename Taxol^{®} by the Bristol-Myers Squibb Company. Paclitaxel (5β,20-Epoxy-1,2α,4,7β,10β,13α-hexahydroxytax-11-en-9-one 4,10-diacetate 2-benzoate 13-ester with (2R,3S)- N-benzoyl-3-phenylisoserine) has the empirical formula C₄₇H₅₁NO₁₄ and a molecular weight of 853.9. It is highly lipophilic in water. Paclitaxel is an antimicrotubule agent that promotes the assembly of microtubles from tubulin dimers and stabilizes microtubules by preventing depolymerization. While not bound by a theory, it is believed that this stability results in the inhibition in the normal dynamic reorganization of the microtubule network that is essential for vital interphase and mitotic cellular functions. Also, paclitaxel is believed to induce abnormal arrays or bundles of microtubules throughout the cell cycle and multiple asters of microtubules during mitosis. Paclitaxel is administered by intravenous injection or by other appropriate infusion techniques.

These and other cytotoxic/cytostatic agents can be administered in the conventional formulations and regimens in which they are known for use alone.

The aryl urea compound can inhibit the enzyme raf kinase. Further, these compounds can inhibit signaling of growth factor receptors. These compounds have been previously described in patent application, serial no. 09/425,228 filed October 26, 1999 which is fully incorporated herein by reference.

The aryl urea compounds can be administered orally, dermally, parenterally, by injection, by inhalation or spray, sublingually, rectally or vaginally in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in polyethyleneglycol, a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal temperature or vaginal temperature and will therefore melt in the rectum or vagina to release the drug. Such materials include cocoa butter and polyethylene glycols.

The Compounds may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of an aryl urea compound in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of an aryl urea compound may be formulated into a lotion or salve.

Suitable solvents for processing transdermal delivery systems are known to those skilled in the art, and include dimethylsulfoxide, lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery systems are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

Its for treating mammalian cancers are described herein. Such kits can be used to treat a patient with a raf kinase stimulated cancer as well as cancers not stimulated through raf kinase. The kit can comprise a single pharmaceutical formulation containing an aryl urea compound and a cytotoxic or cytostatic agent. Alternatively, the kit can comprise an aryl urea compound and a cytotoxic or cytostatic agent in separate formulations. The kit can also include instructions for how to administer the compounds to a patient with cancer in need of treatment. The kit can be used to treat different cancer types which include but are not limited to colon, prostate, leukemia, melanoma, hepatocellular, renal, head and neck, glioma, lung, pancreatic, ovarian, and mammary.

It will be appreciated by those skilled in the art that the particular method of administration will depend on a variety of factors, all of which are routinely considered when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of an aryl urea compound or a pharmaceutically acceptable salt thereof given for a defined number of days, can be ascertained by those skilled in the art using conventional treatment tests.

The usefulness of a combination of an aryl urea compound with a cytotoxic or cytostatic agent is better than could have been expected from conventional knowledge of the effects of using either anticancer agent alone. For example, the combination therapy of an aryl urea compound with the cytotoxic agents irinotecan, gemcitabine, vinorelbine, or paclitaxel has produced at least additive anti-tumor efficacy compared with that produced by administration of either the aryl urea compound or the cytotoxic agents administered alone. Generally, the use of cytotoxic and cytostatic agents in combination with aryl urea compound raf kinase inhibitors will serve to (1) yield better efficacy in reducing the growth of a tumor or even eliminate the tumor as compared to administration of a single chemotherapeutic agent, (2) provide for the administration of lesser amounts of the administered chemotherapeutic agents, (3) provide for a chemotherapeutic treatment that is well tolerated in the patient with less deleterious pharmacological complications resulting from larger doses of single chemotherapies and certain other combined therapies, (4) provide for treating a broader spectrum of different cancer types in mammals, especially humans, (5) provide for a higher response rate among treated patients, (6) provide for a longer survival time among treated patients compared to standard chemotherapy treatments, (7) provide a longer time for tumor progression, and/or (8) yield efficacy and tolerability results at least as good as those of the agents used alone, compared to known instances where other cancer agent combinations produce antagonist effects.

The aryl urea compound can be administered to a patient at a dosage which can range from about 0.1 to about 300 mg/Kg of total body weight. The daily dose for oral administration will preferably be from 0.1 to 300 mg/kg of total body weight. The daily dosage for administration by injection which includes intravenous, intramuscular, subcutaneous and parenteral injection as well as infusion techniques will preferably be from 0.1 to 300 mg/kg of total body weight. The daily vaginal dosage regime will preferably be from 0.1 to 300 mg/kg of total body weight. The daily topical dosage regimen will preferably be from 0.1 to 300 mg administered between one to four times daily. The transdermal concentration will preferably be that required to maintain a daily dose of from 1 to 300 mg/kg. For all the above mentioned routes of administration, the preferred dosage is 0.1 to 300 mg/kg. The daily inhalation dosage regimen will preferably be from 0.1 to 300 mg/kg of total body weight.

The cytotoxic or cytostatic agent can be administered to a patient at a dosage which can range from about 0.1 to about 300 mg/kg of total body weight. Also, the agents can also be administered in conventional amounts routinely used in cancer chemotherapy.

For both the aryl urea compound and the cytotoxic or cytostatic agent, the administered dosage of the compound may be modified depending on any superior or unexpected results which may be obtained as routinely determined with this invention.

The aryl urea compound can be administered orally, topically, parenterally, rectally, by inhalation, and by injection. Administration by injection includes intravenous, intramuscular, subcutaneous, and parenterally as well as by infusion techniques. The aryl urea compound can be present in association with one or more non-toxic pharmaceutically acceptable carriers and if desired other active ingredients. A preferred route of administration for the aryl urea compound is oral administration.

The cytotoxic or cytostatic agent can be administered to a patient orally, topically, parenterally, rectally, by inhalation, and by injection. Administration by injection includes intravenous, intramuscular, subcutaneous, and parenterally as well as by infusion techniques. The agents can be administered by any of the conventional routes of administration for these compounds. The preferred route of administration for the cytotoxic/cytostatic agents using this invention is typically by injection which is the same route of administration used for the agent alone. Any of the cytotoxic or cytostatic agents can be administered in combination with an aryl urea compound by any of the mentioned routes of administration.

For administering the aryl urea compound and the cytotoxic/cytostatic agent, by any of the routes of administration herein discussed, the aryl urea compound can be administered simultaneously with the cytotoxic or cytostatic agent. This can be performed by administering a single formulation which contains both the aryl urea compound and the cytotoxic/cytostatic agent or administering the aryl urea compound and the cytotoxic/cytostatic agents in independent formulations at the same time to a patient.

Alternatively, the aryl urea compound can be administered in tandem with the cytotoxic/cytostatic agent. The aryl urea compound can be administered prior to the cytotoxic/cytostatic agent. For example, the aryl urea compound can be administered once or more times per day up to 28 consecutive days followed by administration of the cytotoxic or cytostatic agent. Also, the cytotoxic or cytostatic agent can be administered first followed by adminstration of the aryl urea compound. The choice of sequence administration of the aryl urea compound relative to the cytotoxic/cytostatic agent may vary for different agents. Also, the cytotoxic or cytostatic agent can be administered using any regimen which is conventionally used for these agents.

In another regimen of administration, the aryl urea compound and the cytotoxic/cytostatic agent can be administered once or more times per day on the day of administration.

Any of the routes and regimens of administration may be modified depending on any superior or unexpected results which may be obtained as routinely determined with this invention.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius and, all parts and percentages are by weight, unless otherwise indicated.

For purposes of the experiments herein described in the Examples, the aryl urea compound (compound A) is a tosylate salt of N-(4-chloro-3-(trifluoromethyl)phenyl)-N'-(4-(2-(N-methylcarbamoyl)-4-pyridyloxy)phenyl)urea.

### EXAMPLES

### Animals

Ncr *nu*/*nu* female mice (Taconic Farms, Germantown, NY) were used for all *in vivo* studies invovling the DLD-1 and NCI-H460 tumor models. Female CB-17 SCID mice (Taconic Farms, Germantown, NY) were used for studies involving the Mia-PaCa-2 tumor model. The mice were housed and maintained within the Comparative Medicine Department at Bayer Corporation, West Haven, CT in accordance with Bayer IACUC, State, and Federal guidelines for the humane treatment and care of laboratory animals. Mice received food and water *ad libitum.*

### Compounds

Compound A (lot 9910071) was used in all studies. Compound A is a dry powder with a color ranging from white to ivory or light yellow. Compound A was stored in the dark until used.

Camptosar® (lot numbers 09FDY and 27FMR) was manufactured by Pharmacia-Upjohn and came supplied as a 20 mg/ml solution. It was stored at room temperature as indicated on the package insert.

Gemzar® (Gemcitabine HCI) was manufactured by Eli Lilly and Company and came supplied as a dry powder. It was stored at room temperature as indicated on the package insert.

Navelbine® (vinorelbine tartrate) was manufactured by GlaxoWellcome, came in as 10mg/ml solution. It was stored in 4°C as indicated on the package.

DOX (Doxorubicin HCl) was manufactured by Bedford Laboratories (lot 110033) and came supplied as a lyophilized red/orange powder. It was stored at 4°C and protected from light.

Gefinitib (ZD1839) (4-(3-chloro-4-fluoroanilino)7-methoxy-6-(3-morpholinopropoxy)quinazoline was synthesized by Albany Medical Research (Syracuse, NY). ZD1839 was stored in the dark at room temperature until used.

### Vehicles

Cremophor EL /Ethanol (50:50) (Sigma Cremophor EL Cat.# C-5135; 500g, 95% Ethyl Alcohol), was prepared as a stock solution, wrapped with aluminum foil, and stored at room temperature. Compound A was formulated at 4-fold (4X) of the highest dose in this Cremophor EL/Ethanol (50:50) solution. This 4X stock solution was prepared fresh every three days. Final dosing solutions were prepared on the day of use by dilution to 1X with endotoxin screened distilled H₂O (GIBCO, Cat.# 15230-147) and mixed by vortexing immediately prior to dosing. Lower doses were prepared by dilution of the 1X solution with Cremophor EL/Ethanol/water (12.5:12.5:75). The vehicle for Camptosar® and Gemzar® was 0.9% saline and the vehicle for Navelbine® was D5W. All vehicles and compound solutions were stored at room temperature and wrapped in foil.

### Tumor Lines

The DLD-1 human colon carcinoma and the MiaPaCa-2 human pancreatic carcinoma were obtained from the American Type Tissue Culture Collection Repository. The MX-1 human mammary tumor was obtained from the NCI tumor repository. Tumors were maintained as a serial in vivo passage of s.c. fragments (3 x 3 mm) implanted in the flank using a 12 gauge trocar. A new generation of the passage was initiated every three or four weeks.

The NCI-H460 and A549 human non-small-cell lung carcinoma lines were obtained from the American Type Tissue Culture Collection Repository. The NCI-H460 cells were maintained and passaged in vitro using DMEM (GIBCO cat. # 11995-065: 500 mls) supplemented with 10% heat inactivated fetal bovine serum (JRH Biosciences cat.# 12106-500M), 2mM L-glutamine (GIBCO cat. # 25030-81), 10mM HEPES buffer (GIBCO cat # 15630-080) and penicillin-streptomycin (GIBCO cat. # 15140-122: 5 mls/ 50 mls DMEM). The A549 cells were maintained and passaged using RPMI 1640 media (GIBCO cat.# 11875-085: 1000ml) supplemented with 10% heat-inactivated fetal bovine serum (JRH Biosciences cat.# 12106-500M). All cells were maintained at 37°C and 5% CO₂ in a Fisher Scientific 610 CO₂ incubator.

### Tumor Xenograft Experiments

Female mice were implanted s.c. with DLD-1, MX-1 or Mia-PaCa-2 tumor fragments from an *in vivo* passage. Studies with the NCI-H460 and A549 cells were initiated by harvesting cells from an *in vitro* culture by adding Trypsin-EDTA (GIBCO cat#25200-056) for 2 minutes followed by centrifugation of the cells into a pellet and resuspension in HBSS (GIBCO cat# 14025-092) to a final cell count of 3-5 x 10⁷ viable cells/ml. A volume of 0.1ml of the cell suspension was injected s.c. in the right flank of each mouse. All treatment was initiated when all mice in the experiment had established tumors ranging in size from 100 to 150 mg. The general health of mice was monitored and mortality was recorded daily. Tumor dimensions and body weights were recorded twice a week starting with the first day of treatment. Animals were euthanized according to Bayer IACUC guidelines. Treatments producing greater than 20% lethality and/or 20% net body weight loss were considered 'toxic'.

Tumor weights were calculated using the equation (*l x w*²)/2, where *l* and w refer to the larger and smaller dimensions collected at each measurement. In each experiment, an evaluation endpoint was selected such that the median time for the tumors in the control group to attain that size was slightly greater than the duration of treatment. Anti-tumor efficacy was measured as the incidence of complete regressions (CR) defined as tumors that are reduced to below the limit of measurement (3 mm) in both length and width, partial regressions (PR) defined as tumors that are reduced by more than 50% but less than 100% of their initial size, and percent tumor growth suppression (%TGS). TGS is calculated by the equation [(T-C)/C] * 100, where T and C represent the times for the median tumors in the treated (T) and untreatred control (C) groups, respectively, to attain the evaluation size for that experiment.

### Results

### Combination of compound A and cytotoxic/cytostatic agents

The most intensive combination chemotherapy anticipated in the clinical development of compound A for the treatment of cancer would involve daily administration of compound A administered throughout the period of time encompassing the intermittent administration of cytotoxic/cytostatic agents such as e.g., Camptosar®, Gemzar®, Navelbine®, or DOX that constitute the current clinical practice with each of these agents. In order to explore the potential interactions of these agents, we modeled this anticipated clinical schedule in our preclinical model by superimposing the schedules of the individual agents (qd x 9 for compound A and q4d x 3 for Camptosar®, Gemzar®, Navelbine®, or DOX) with both therapies in each experiment starting on the same day. An alternative schedule of combination chemotherapy would consist of daily administration of compound A throughout the period of time encompassing the continuous administration of cytostatic agents such as Iressa®. In order to explore the potential interactions of these agents, the preclinical model was established by superimposing the schedules of the individual agents (qd x 9 or 10 for both compound A and Iressa^{®}). These schedules are termed 'Concurrent Therapy'. Each study consisted of an untreated control group of 10-20 animals and treated groups of 10 mice per group.

### Example 1

In the first study, Camptosar® was administered i.p at 40 mg/kg/dose. Compound A was administered p.o. on a qd x 9 schedule at 80 mg/kg/dose. All treatment was initiated on Day 7 post-implant when all animals had small but established DLD-1 human colon tumor xenografts averaging 108 mg in size. Control tumors grew progressively in all animals with an average doubling time of 4.4 days. The evaluation endpoint used to calculate the growth delay parameters was time to three mass doublings. The median time for the tumors in the untreated control group to attain that size was 10.4 days.

Camptosar® was well tolerated as a single agent with minimal weight loss and no lethality. The 40 mg/kg dose level produced a TGS of 71% with no complete or partial tumor regressions.

Compound A was also well tolerated as a single agent producing no significant weight loss and no lethality at 80 mg/kg/dose. Compound A produced a TGS of 100%.

There was no increase in weight loss and no lethality associated with the combination of Camptosar® with compound A. The anti-tumor efficacy of the concurrent therapy was at least additive producing a 229% TGS. This was associated with 3 PR's.

### Example 2

The second study evaluated Gemzar®, administered i.p at 120 mg/kg/dose on a q4d x 3 schedule and compound A, administered p.o. on a qd x 9 schedule at 40 mg/kg/dose. All treatment was initiated on Day 7 post-implant when all animals had small but established MiaPaCa-human pancreatic tumor xenografts averaging 108 mg in size. Control tumors grew progressively in all animals with an average doubling time of 4.1 days. The evaluation endpoint used to calculate the growth delay parameters was time to two mass doublings. The median time for the tumors in the untreated control group to attain that size was 5.8 days.

Gemzar® was well tolerated as a single agent with no weight loss and no lethality. This dose level produced a TGS of 154% with no complete or partial tumor regressions. Compound A was also well tolerated as a single agent producing no significant weight loss and no lethality at the 80 mg/kg dose level. Compound A produced TGS of 112%.There was no increase in weight loss and no lethality associated with the combination of Gemzar® with Compound A. The anti-tumor efficacy of the concurrent therapy of 120 mg/kg Gemzar and 40 mg/kg Compound A was at least additive producing a 222% TGS. This was associated with 2 PR's.

### Example 3

The third example demonstrates the effect of the combination of Compound A, administered p.o. on a qd x 9 schedule at 40 mg/kg/dose and Navelbine®, administered i.v. on a q4d x 3 schedule at 6.7 mg/kg/dose. All treatment was initiated on Day 6 post-implant when all animals had small but established NCI-H460 human non-small cell lung tumor xenografts averaging 100 mg in size. Control tumors grew progressively in all animals with an average doubling time of 3.1 days. The evaluation endpoint used to calculate the growth delay parameters was time to three mass doublings. The median time for the tumors in the untreated control group to attain that size was 7.4 days. The 6.7 mg/kg dose level of Navelbine was an approximate maximum tolerated dose producing an average 19% weight loss during the treatment period as a single agent. This was associated with a 32% TGS. Compound A was well tolerated with no significant weight loss and produced a TGS of 104%. The combination of these treatments was well tolerated with no lethality and an average weight loss of 14% (less than that produced by Navelbine alone). The antitumor efficacy of this combination was also approximately additive with a TGS of 133%.

### Example 4

The fourth example demonstrates the effect of the combination of Compound A, administered p.o. on a qd x 9 schedule at 40 mg/kg/dose and DOX, administered i.v. on a q4d x 3 schedule at 4 mg/kg/dose. All treatments were initiated on Day 6 post-implant when all animals had small but established tumors averaging 66 mg in size. Control tumors grew progressively in all animals with an average doubling time of 3.7 days. The evaluation endpoint used to calculate the growth delay parameters was time to four mass doublings. The median time for the tumors in the untreated control group to attain that size was 14.5 days. The 4 mg/kg dose level of DOX was well tolerated producing an average 5% weight loss during the treatment period as a single agent. This was associated with a 43% TGS. Compound A was well tolerated with no significant weight loss and produced a TGS of 46%. The combination of these treatments was tolerated with no lethality and an average weight loss of 12%. The antitumor efficacy of this combination was also approximately additive with a TGS of 133%.

### Example 5

The fifth example demonstrates the effect of the combination of Compound A, administered p.o. on a qd x 9 schedule at 80 mg/kg/dose and Gefinitib (Iressa^{®}), administered p.o. on a qd x 9 schedule at 150 mg/kg/dose. All treatment was initiated on Day 15 post-implant when all animals had small but established A549 human non-small cell lung tumor xenografts averaging 110 mg in size. Control tumors grew progressively in all animals with an average doubling time of 10.5 days. The evaluation endpoint used to calculate the growth delay parameters was time to one mass doubling. The 150 mg/kg dose level of Iressa^{®} was well tolerated producing no weight loss and no lethality during the treatment period as a single agent. This treatment was associated with a 101% TS and 1 PR. Compound A was also well tolerated as a single agent with no weight loss or lethality and produced a TGS of 218% with 1 CR and 2 PRs. The combination of these treatments was tolerated with one non-specific death out of 10 mice and an average 10% weight loss. The antitumor efficacy of this combination was approximately additive with a TGS of 314%. This treatment also produced 6 CR's and 3 PR's.

The preceding examples can be repeated with similar success by substituting the generically or specifically described reactants and/or operating conditions of this invention for those used in the preceding examples.

## Claims

1. Use of an aryl urea compound and doxorubicin or a pharmaceutically acceptable salt thereof for the preparation of a medicament for the treatment of a hepatocellular cancer, wherein said aryl urea compound is a tosylate salt of N-(4-chloro-3-(trifluoromethyl)phenyl-N'-(4-(2-(N-methylcarbamoyl)-4-pyridyloxy)phenyl)urea.

2. The use of claim 1, wherein said composition is administered in a therapeutically effective amount to a patient in need thereof by oral delivery or by intravenous injection or infusion.

3. The use of claim 1, wherein said composition is administered in a therapeutically effective amount to a patient in need thereof in the form of a tablet, a liquid, a topical gel, an inhaler or in the form of a sustained release composition.

4. The use of claim 1, wherein the aryl urea compound is administered to a patient at an oral, intravenous, intramuscular, subcutaneous or parenteral dosage which can range from about 0.1 to about 300 mg/kg of total body weight.

5. The use of claim 1, wherein the doxorubicin is administered to a patient at an oral, intravenous, intramuscular, subcutaneous or parenteral dosage which can range from about 0.1 to about 300 mg/kg of total body weight.

6. The use of claim 1 for inhibiting the proliferation of cancer cells in a patient.

7. The use of claim 1, wherein said aryl urea compound is administered simultaneously with doxorubicin or a pharmaceutically acceptable salt thereof.

8. The use of claim 7, wherein said aryl urea compound and doxorubicin or a pharmaceutically acceptable salt thereof are administered in the same formulation or in separate formulations.

9. The use of claim 1, wherein said aryl urea compound is administered sequentially with doxorubicin or a pharmaceutically acceptable salt thereof, in any order.

10. The use of claim 1, wherein said aryl urea compound is administered in tandem with doxorubicin or a pharmaceutically acceptable salt thereof wherein said aryl urea compound can be administered to a patient once or more per day for up to 28 consecutive days with the concurrent or intermittent administration of doxorubicin or a pharmaceutically acceptable salt thereof over the same total time period.

## Patentansprüche

1. Verwendung von einer Arylharnstoffverbindung und Doxorubicin oder eines pharmazeutisch akzeptablen Salzes davon zur Herstellung eines Medikaments zur Behandlung von Leberzellkrebs, wobei die Arylharnstoffverbindung ein Tosylatsalz von N-(4-chlor-3-(trifluormethyl)phenyl-N'-(4-(2-(N-methylcarbamoyl)-4-pyridyloxy) phenyl)Harnstofif ist.

2. Verwendung nach Anspruch 1, wobei die Zusammensetzung in einer therapeutisch wirksamen Menge an einen Patienten, der es benötigt, durch orale Abgabe oder mittels intravenöser Injektion oder Infusion verabreicht wird.

3. Verwendung nach Anspruch 1, wobei die Zusammensetzung in einer therapeutisch wirksamen Menge an einen Patienten, der es benötigt, in der Form einer Tablette, einer Flüssigkeit, eines topischen Gels, eines Inhalators oder in Form einer Zusammensetzung mit verzögerter Freisetzung verabreicht wird.

4. Verwendung nach Anspruch 1, wobei die Arylharnstoffverbindung an einen Patienten mit einer oralen, intravenösen, intramuskulären, subkutanen oder parenteralen Dosierung, die zwischen etwa 0,1 bis 300 mg/kg des Gesamtkörpergewichts variieren kann, verabreicht wird.

5. Verwendung nach Anspruch 1, wobei Doxorubicin an einen Patienten mit einer oralen, intravenösen, intramuskulären, subkutanen oder parenteralen Dosierung, die zwischen etwa 0,1 bis 300 mg/kg des Gesamtkörpergewichts variieren kann, verabreicht wird.

6. Verwendung nach Anspruch 1 zur Inhibierung der Proliferation von Krebszellen in einem Patienten.

7. Verwendung nach Anspruch 1, wobei die Arylharnstoff-verbindung gleichzeitig mit Doxorubicin oder einem pharmazeutisch akzeptablen Salz davon verabreicht wird.

8. Verwendung nach Anspruch 7, wobei die Arylharnstoffverbindung und Doxorubicin oder ein pharmazeutisch akzeptables Salz davon in der gleichen Formulierung oder in separaten Formulierungen verabreicht werden.

9. Verwendung nach Anspruch 1, wobei die Arylharnstoffverbindung sequenziell mit Doxorubicin oder einem pharmazeutisch akzeptablen Salz davon in beliebiger Reihenfolge verabreicht wird.

10. Verwendung nach Anspruch 1, wobei die Arylharnstoffverbindung als Tandem mit Doxorubicin oder einem pharmazeutisch akzeptablen Salz davon verabreicht wird, wobei die Arylharnstoffverbindung einem Patienten einmal oder mehrmals täglich für bis zu 28 aufeinander folgende Tage mit der konkurrierenden oder intermittierenden Verabreichung von Doxorubicin oder eines pharmazeutisch akzeptablen Salzes davon über die gleiche Gesamtdauer verabreicht werden kann.

## Revendications

1. Utilisation d'un composé urée aryle et de doxorubicine ou d'un sel pharmaceutiquement acceptable de celle-ci pour la préparation d'un médicament destiné au traitement d'un cancer hépatocellulaire, dans laquelle ledit composé urée aryle est un sel de tosylate de la N-(4-chloro-3-(trifluorométhyl)phényl-N'-(4-(2-(N-méthylcarbamoyl)-4-pyridyloxy)phényl)urée.

2. Utilisation selon la revendication 1, dans laquelle ladite composition est administrée en une quantité efficace d'un point de vue thérapeutique à un patient ayant besoin de celle-ci par voie orale ou par perfusion ou injection intraveineuse.

3. Utilisation selon la revendication 1, dans laquelle ladite composition est administrée en une quantité efficace d'un point de vue thérapeutique à un patient ayant besoin de celle-ci sous la forme d'un comprimé, d'un liquide, d'un gel topique, d'un inhalant ou sous la forme d'une composition à libération prolongée.

4. Utilisation selon la revendication 1, dans laquelle le composé urée aryle est administré à un patient à une dose orale, intraveineuse, intramusculaire, sous-cutanée ou parentérale qui peut être située dans la plage d'environ 0,1 à environ 300 mg/kg de poids corporel total.

5. Utilisation selon la revendication 1, dans laquelle la doxorubicine est administrée à un patient à une dose orale, intraveineuse, intramusculaire, sous-cutanée ou parentérale qui peut être située dans la plage d'environ 0,1 à environ 300 mg/kg de poids corporel total.

6. Utilisation selon la revendication 1, destinée à inhiber la prolifération de cellules cancéreuses chez un patient.

7. Utilisation selon la revendication 1, dans laquelle ledit composé urée aryle est administré simultanément avec de la doxorubicine ou un sel pharmaceutiquement acceptable de celle-ci.

8. Utilisation selon la revendication 7, dans laquelle ledit composé urée aryle et la doxorubicine ou un sel pharmaceutiquement acceptable de celle-ci sont administrés dans la même formulation ou dans des formulations séparées.

9. Utilisation selon la revendication 1, dans laquelle ledit composé urée aryle est administré de manière séquentielle avec de la doxorubicine ou un sel pharmaceutiquement acceptable de celle-ci, dans n'importe quel ordre.

10. Utilisation selon la revendication 1, dans laquelle ledit composé urée aryle est administré en tandem avec de la doxorubicine ou un sel pharmaceutiquement acceptable de celle-ci, dans laquelle ledit composé urée aryle peut être administré à un patient une fois ou plusieurs fois par jour pendant jusqu'à 28 jours consécutifs avec l'administration concomitante ou intermittente de doxorubicine ou d'un sel pharmaceutiquement acceptable de celle-ci sur la même période de temps totale.
